# EUROPEAN PATENT APPLICATION

(11) **EP 4 246 521 A1**
(43) Date of publication of application: **20.09.2023**
(21) Application number: 21892342.3
(22) Date of filing: 11.11.2021
(51) Int. Cl.: G16B 40/20, G16B 15/30, G16B 25/10, C12Q 1/68

(54) **TARGET ANTIGEN DISCOVERY METHOD AND ANALYSIS APPARATUS FOR CHIMERIC ANTIGEN RECEPTOR**

(30) Priority: 11.11.2020 KR 20200149990
(71) Applicant: Korea Advanced Institute of Science and Technology, Daejeon 34141 (KR)
(72) Inventor: CHOI, Jung Kyoon, Daejeon 34141 (KR); KWON, Joon Ha, Daejeon 34141 (KR)
(74) Representative: Ostertag & Partner Patentanwälte mbB
(86) International application number: PCT/KR2021/016455
(87) International publication number: WO 2022/103175

(57) **Abstract**

A method for discovering a target antigen for a chimeric antigen receptor comprises the steps of: obtaining, by an analysis apparatus, expression information of genes of tumor cells in single cell units; extracting, by the analysis apparatus, from among the genes, genes of candidate antigens having an expression level of a reference value or more; determining, by the analysis apparatus, all possible gene combinations that can be constituted by the genes of the candidate antigens; and determining, by the analysis apparatus, a target gene combination in which at least one gene included therein is expressed, among the gene combinations.

## Description

### [Technical Field]

The technology described below relates to a method of discovering an antigen for a chimeric antigen receptor (CAR).

### [Background Art]

Immune cell therapy for cancer is a therapeutic method of removing tumor cells using *ex vivo* cultured T cells. A CAR is a binding site recognizing an antigen that is designed to bind to an active domain in T cells. CAR-T is T cell therapy that is applied to T cells. On the other hand, CAR-NK is cell therapy that is applied to natural killer (NK) cells.

### [Disclosure]

### [Technical Problem]

For CAR-T and CAR-NK, effective target antigen discovery is very important. In the target antigen discovery, coverage which indicates whether CAR-T and CAR-NK act on a large number of tumor cells, and specificity which indicates whether cell toxicity applied to normal cells is minimized and they act only on tumor cells are critical indicators.

The technology described below is directed to providing a method of discovering a target antigen to which a chimeric antigen receptor (CAR) binds for treatment of a specific tumor.

### [Technical Solution]

One aspect of the technology described below provides a method of discovering a target antigen for a CAR, which includes acquiring expression information of genes of tumor cells in a single cell unit by an analysis apparatus, extracting genes of candidate antigens having an expression level of a reference value or higher from the genes by the analysis apparatus, determining gene combinations which can consist of the genes of the candidate antigens by the analysis apparatus, determining a target gene combination in which at least one gene included in the combination is expressed among the gene combinations by the analysis apparatus, and determining at least one of the target gene compositions as a gene combination for a target antigen by the analysis apparatus.

Another aspect of the technology described below provides an analysis apparatus for CAR design, which includes an input unit for receiving expression data of genes in tumor cells of a sample in a single cell unit, a storage unit for storing a program that determines an antigen recognized by a CAR using the expression information of genes in tumor cells, and an arithmetic unit for determining a target gene combination or target gene for a target antigen of tumor cells in the sample recognized by the CAR from gene combinations that can consist of a plurality of genes among the genes of the candidate antigens determined from the expression data of the genes.

### [Advantageous Effects]

According to the technology to be described below, an antigen having wide coverage and high specificity in the design of a chimeric antigen receptor (CAR) can be discovered.

### [Description of Drawings]

FIG. 1 illustrates an example of antigen expression patterns in single cells and a cell population.
FIG. 2 illustrates an example of a system for discovering a target antigen for a chimeric antigen receptor (CAR).
FIG. 3 illustrates one example of a flowchart illustrating a process of discovering a target antigen for a CAR.
FIG. 4 illustrates another example of a flowchart illustrating a process of discovering a target antigen for a CAR.
FIG. 5 illustrates still another example of a flowchart illustrating a process of discovering a target antigen for a CAR.
FIG. 6 illustrates an example of a process of discovering a target antigen by an analysis apparatus.
FIG. 7 illustrates an experimental result of the gene expression patterns of tumor cells and normal cells in a colorectal cancer sample and a lung cancer sample.
FIG. 8 illustrates an experimental result for a gene combination in which one or more genes are expressed in a lung cancer sample.
FIG. 9 illustrates an experimental result for a gene combination in which one or more genes are expressed in a colorectal cancer sample.
FIG. 10 illustrates an experimental result for a gene combination in which two or more genes are expressed in a lung cancer sample.
FIG. 11 illustrates an experimental result for a gene combination in which two or more genes are expressed in a colorectal cancer sample.
FIG. 12 illustrates an experimental result for a tumor cell-specific gene combination in a lung cancer sample.
FIG. 13 illustrates an experimental result for a tumor cell-specific gene combination in a colorectal cancer sample.
FIG. 14 illustrates an example of an analysis apparatus for discovering an antigen for a CAR.
FIG. 15 illustrates an example of the process of identifying a gene combination that discriminates tumor cells and normal cells using large-scale single cell transcriptome data.
FIG. 16 illustrates a result of comparing patterns of RNA expression and protein expression against an antigen of ovarian cancer cells.
FIG. 17 illustrates another example of a process of discovering a target antigen for a CAR.

### [Modes of the Invention]

The technology described below may have various modifications and various examples, and thus specific examples are illustrated in the drawings and described in detail in the detailed description. However, it should be understood that the technology described below is not limited to specific embodiments, and includes all modifications, equivalents or alternatives within the spirit and technical scope of the technology described below.

The terms "first," "second," "A," and "B" may be used to describe various components, but the components should not be limited by these terms. The terms are used only to distinguish one component from another component. For example, without departing from the scope of rights of the technology described below, a first component may be referred to as a second component, and similarly, a second component may also be referred to as a first component. The term "and/or" encompasses a combination of a plurality of related items described herein, or any one of a plurality of the related items described herein.

It should be understood that singular expressions used herein include plural expressions unless clearly interpreted otherwise in the context. In the specification, it should be understood that the term "include" or "have" is intended to indicate the presence of a characteristic, a number, a step, an action, a component or a part, which is described, or a combination thereof, but does not preclude the possibility of the presence or addition of one or more other characteristics, numbers, steps, actions, components, parts or a combination thereof.

Prior to the detailed description of the drawings, it should be clarified that the division of components in the specification is merely the classification of components depending on the main function of each component. That is, two or more components to be described below may be combined into one component, or divided into two or more segments by subdivided function. In addition, it is obvious that each component to be described below may additionally perform some or all of the functions of the functions of other components, in addition to its main function, or some of the main functions of the respective components may be exclusively performed by other components.

In addition, during the accomplishment of a method or an operating method, procedures comprising the method may be performed in a different order from the specified order unless clearly and particularly stated otherwise in the context. That is, the procedures may be performed in the same order as specified, performed substantially simultaneously, or performed in a reverse order.

Terms used below will be described.

An antigen is a material that induces an immune response.

Hereinafter, an antigen is a site in tumor cells, which is recognized by a chimeric antigen receptor (CAR). Antigens may be tumor-specific antigens (TSAs) which are not expressed in normal cells but expressed in tumor cells, or tumor-associated antigens (TAAs) which are expressed in normal cells and also shown at a particularly high frequency in cancer cells due to an antigenic change in the cancerization of cells, but the present invention is not limited thereto.

A CAR mainly uses a single-chain variable fragment (scFv). Accordingly, an antigen may include a protein expressed on the surface of a tumor cell, a sugar chain, and a sugar lipid.

A target antigen described below is defined as a target that is recognized by a CAR for tumor cell death. The technology described below is to discover a target antigen.

A sample is a single cell or multiple cells, a cell fragment, or a body fluid taken from a subject to be analyzed.

Genetic data or genetic information is genetic information produced by analyzing a sample. For example, genetic data may include deoxyribonucleic acid (DNA) and ribonucleic acid (RNA), which are obtained from cells, tissue, etc., a base sequence, genetic expression data, genetic standard genetic data obtained from a protein, genetic variation with standard genetic data, DNA methylation, etc., which are obtained from a protein. Genetic data of cells described below is information related to an antigen mainly expressed on a cell surface. Accordingly, genetic data may include information such as a genome, a transcriptome, and a proteome. That is, genetic data is data from which the degree of specific gene expression (particularly, antigen expression on a cell surface) can be confirmed regardless of its type. Genetic data is assumed to be digital data that can be processed by a computer apparatus.

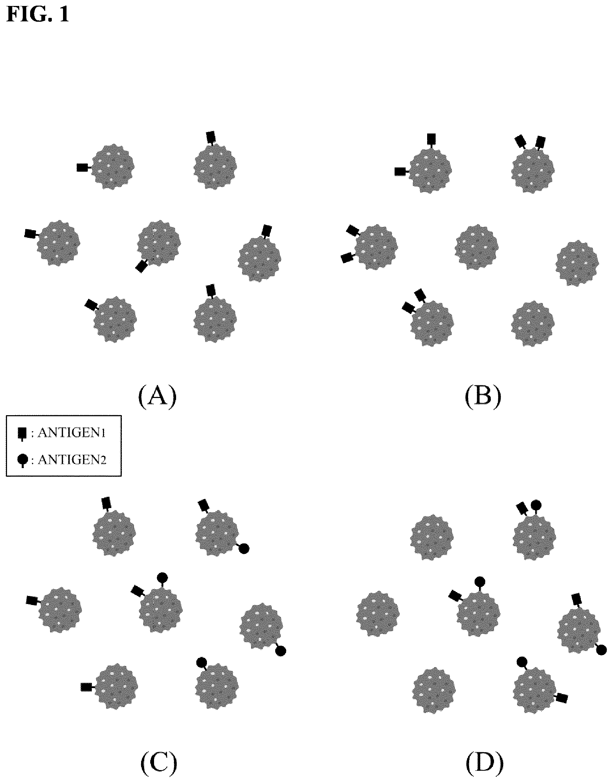

FIG. 1 is an example of antigen expression patterns in single cells and a cell population.

FIGS. 1A and 1B illustrate the state in which a single antigen is expressed in tumor cells. In FIGS. 1A and 1B, the single antigen is antigen 1. FIG. 1A is an example of expression observed at a single cell level, and FIG. 1B is an example of expression observed in a cell population. Referring to FIG. 1A, antigen 1 has 100% coverage for a single cell. Referring to FIG. 1B, the antigen 1 has 4/7 coverage for a cell population. Accordingly, when CAR-T is designed as a CAR recognizing the antigen 1 confirmed to be expressed based on the result of FIG. 1B, the corresponding CAR-T attacks only a specific clone in tumor cells and prevents other clones in which the corresponding antigen is not expressed from being removed. The same is true of CART-NK.

FIGS. 1C and 1D illustrate the state in which a plurality of antigens are expressed in tumor cells. In FIGS. 1A and 1B, the plurality of antigens include antigen 1 and antigen 2. FIG. 1C is an example of observing expression to a single cell level, and FIG. 1D is an example of observing expression in a cell population. Referring to FIG. 1C, the antigens 1 and 2 have 100% coverage for a single cell. Referring to FIG. 1D, the antigens 1 and 2 have 4/7 coverage for a cell population. Accordingly, when CAR-T is designed as a CAR recognizing the antigen 1 confirmed to be expressed based on the result of FIG. 1D, the corresponding CAR-T attacks only a specific clone in tumor cells and prevents other clones in which the corresponding antigen is not expressed from being removed. The same is true of CART-NK.

In the end, it is difficult to design a CAR with high coverage when the expression of an antigen is not verified based on a single cell as described above, but decided to a population level according to a conventional method. To design a CAR with wide coverage and high specificity, effective target antigen discovery is important. Hereinafter, a new methodology for target antigen discovery will be described.

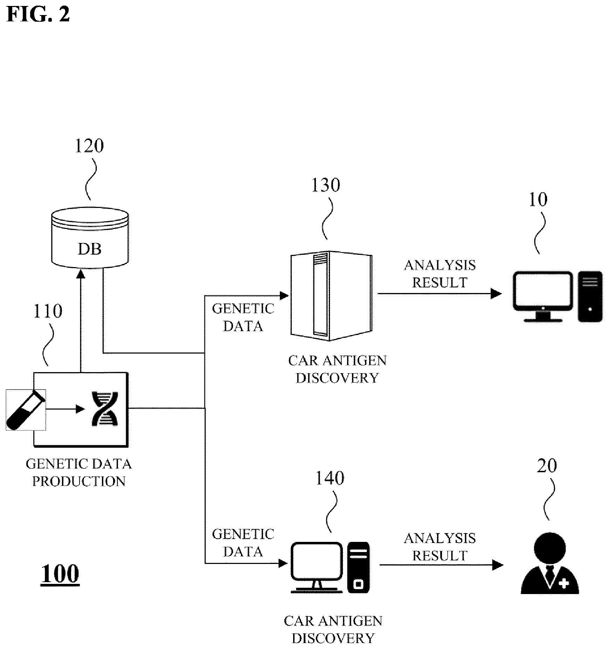

FIG. 2 is an example of a target antigen discovery system 100 for a CAR. Analysis apparatuses 130 and 140 are apparatuses for discovering a target antigen for a CAR. In FIG. 2, the analysis apparatuses are illustrated in the form of a server 130 and a computer terminal 140. The server 130 may provide a service of discovering a target antigen on a network. The computer terminal 140 may be connected to a network or discover a target antigen as a discrete apparatus. Meanwhile, the analysis apparatuses 130 and 140 may be realized in various forms.

A gene analysis apparatus 110 produces genetic data of a sample by analyzing the sample. A gene database (DB) 120 may store and manage the produced genetic data. The genetic data may include a genetic sequence and gene expression information of the sample. The genetic data includes the expression information on antigen genes on the surface of a tumor cell. The genetic data includes information on the expression levels of antigen genes. On the other hand, each of the plurality of genes corresponds to one antigen.

The gene DB 120 may be an open DB possessing the results of experiments conducted by multiple researchers. Meanwhile, the gene DB 120 may possess genetic data of the sample of a specific patient. In this case, the analysis apparatuses 130 and 140 may discover a patient-specific antigen.

The analysis apparatuses 130 and 140 analyze genetic data to discover a target antigen for a CAR. A process of discovering a target antigen by the analysis apparatuses 130 and 140 will be described.

Users 10 and 20 may confirm the results of discovering a target antigen for a specific tumor or specific patient. The user 10 may log in to a server 130 through a user terminal (PC, smartphone or the like) to confirm the analysis result accomplished by the server 130. The user 20 may confirm a target antigen discovery result through the computer terminal 140 used by the user 20.

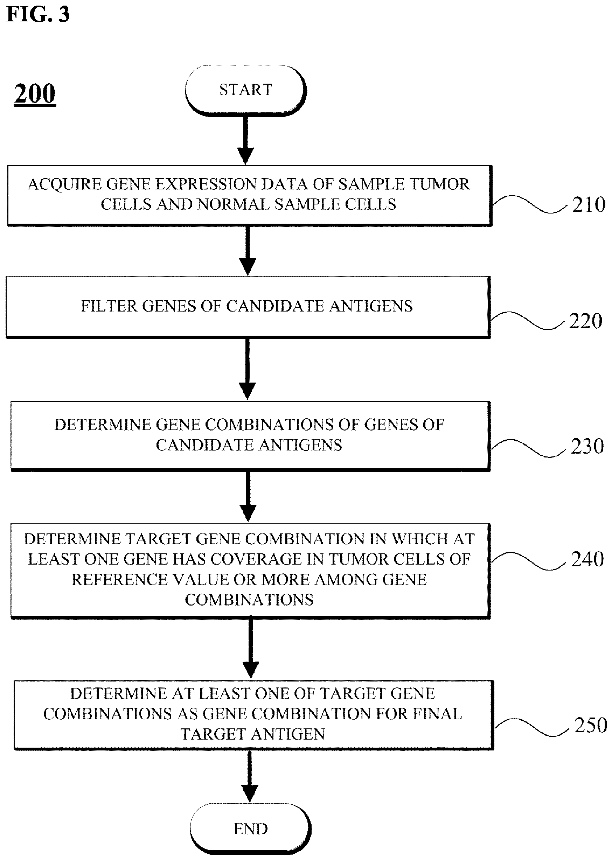

FIG. 3 is one example of a flowchart illustrating a process (200) of discovering a target antigen for a CAR.

The analysis apparatus acquires gene expression information of sample tumor cells (210). Here, the sample may be samples of a specific type of tumor (e.g., lung cancer) collected from multiple persons. Alternatively, the sample may be cells taken from a specific patient. The analysis apparatus may also acquire gene expression information of normal sample cells (210). Information on normal cells may be used for specificity evaluation. Here, tumor cells and normal cells may be cells acquired from various cell populations, rather than a single clone.

The genetic data of tumor cells and/or normal cells collected by the analysis apparatus is referred to as source data. The source data includes gene expression information in a discrete cell unit through single cell analysis. Tumor cells included in the source data may also be referred to as a tumor cell group. In addition, the normal cells included in the source data may also be referred to as a normal cell group.

The analysis apparatus may filter effective data from the source data (220). The analysis apparatus may select genes of candidate antigens from the source data (220). The candidate antigens correspond to antigens that are a population for discovering a target antigen. A gene of a candidate antigen is a gene directly or indirectly involved in the expression of a corresponding antigen. The gene(s) involved in expression of a candidate antigen may be referred to as candidate gene(s). In addition, a set of genes involved in expression of each candidate antigen may be referred to as a candidate gene group.

For the selection of a candidate gene, a variety of criteria may be used. (i) An analysis apparatus may select a gene expressed at a certain rate or more as a candidate gene in cells included in a tumor cell group based on the tumor cell group. For example, the analysis apparatus may select a gene for an antigen expressed at 1% or more in the tumor cell group as a candidate gene. (ii) The analysis apparatus may select a gene that has a certain difference in expression pattern in tumor cells and normal cells as a candidate gene. For example, the analysis apparatus may analyze a differential expression gene (DEG) in tumor cells, compared to normal cells, as a candidate gene. The analysis apparatus may select a gene whose expression level in tumor cells is the same as or higher than the reference value compared to that of normal cells. (iii) Further, the analysis apparatus may select a candidate gene by combining the criterion (i) and the criterion (ii). For example, the analysis apparatus may select a gene differentially expressed greater than a certain standard in tumor cells among the genes for an antigen expressed 1% or greater in a tumor cell group as a candidate gene.

The analysis apparatus determines all combinations that can consist of candidate genes based on the filtered genes of the candidate antigens (candidate genes) (230). The analysis apparatus may determine all available combinations mathematically according to the number of cases. Alternatively, the analysis apparatus may determine a possible gene combination for genes substantially expressed simultaneously considering the expression relationship of genes.

The analysis apparatus confirms whether at least one gene included in each gene combination is expressed greater than the reference rate in tumor cells. The analysis apparatus determines a target gene combination based on a rate (coverage) of cells in which at least one gene included in each combination is expressed among all tumor cells (240). For example, when tumor cells in which at least one gene included in the combination is expressed account for 60% or more of all of the tumor cells, the analysis apparatus may determine the corresponding combination as a target gene combination. The target gene combination includes a gene involved in the expression of a target gene recognized by a CAR. At least one gene included in the gene combination is involved in the expression of one target antigen.

The analysis apparatus determines the final gene combination for a target antigen among the target gene combinations (250). (i) The analysis apparatus may determine at least one of the top-rated gene combinations in terms of coverage from the target gene combinations as the final gene combination. (ii) The analysis apparatus may determine at least one of the top-rated multiple gene combinations in terms of coverage from the target gene combinations, in which coverage in normal cells is less than the reference value, as the final gene combination.

In FIG. 3, the analysis apparatus discovers a target antigen based on at least one antigen expressed in tumor cells. That is, when at least one gene in a gene combination has coverage the same as or greater than the reference value in tumor cells, the analysis apparatus may determine that the corresponding gene is involved in an effective candidate antigen. In other words, when at least one antigen is expressed at a certain rate or more in tumor cells, the analysis apparatus determines the corresponding antigen as a target antigen.

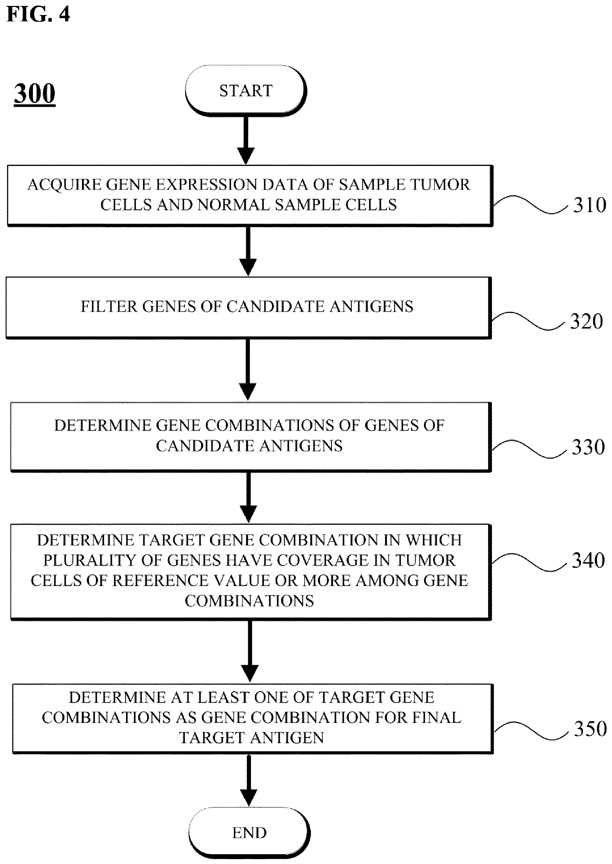

FIG. 4 is another example of a flowchart illustrating a process (300) of discovering a target antigen for a CAR.

An analysis apparatus acquires gene expression information of sample tumor cells (310). Here, the sample may be samples of a specific type of tumor (e.g., lung cancer) collected from multiple persons. Alternatively, the sample may be cells taken from a specific patient. The analysis apparatus may also acquire gene expression information of normal sample cells (310). Information on normal cells may be used for specificity evaluation. Here, tumor cells and normal cells may be cells acquired from various cell populations, rather than a single clone.

The analysis apparatus may filter effective data from the source data (320). The analysis apparatus may select genes of candidate antigens (candidate genes) from the source data (320).

For the candidate gene selection, a variety of criteria may be used. (i) An analysis apparatus may select a gene expressed at a certain rate or more as a candidate gene in cells included in a tumor cell group based on the tumor cell group. For example, the analysis apparatus may select a gene for an antigen expressed at 1% or more in the tumor cell group as a candidate gene. (ii) The analysis apparatus may select a gene that has a certain difference in expression pattern in tumor cells and normal cells as a candidate gene. For example, the analysis apparatus may analyze a differential expression gene (DEG) in tumor cells, compared to normal cells, as a candidate gene. The analysis apparatus may select a gene whose expression level in tumor cells is the same as or higher than the reference value compared to that of normal cells. (iii) Further, the analysis apparatus may select a candidate gene by combining the criterion (i) and the criterion (ii). For example, the analysis apparatus may select a gene differentially expressed greater than a certain standard in tumor cells among the genes for an antigen expressed 1% or greater in a tumor cell group as a candidate gene.

The analysis apparatus determines all combinations that can consist of candidate genes based on the filtered genes (candidate genes) of the candidate antigens (candidate genes) (330). The analysis apparatus may determine all available combinations mathematically according to the number of cases. Alternatively, the analysis apparatus may determine a possible gene combination for genes expressed substantially simultaneously considering the expression relationship of genes.

The analysis apparatus confirms whether at least one gene included in each gene combination is expressed greater than the reference rate in tumor cells. The analysis apparatus determines a target gene combination based on coverage of cells in which at least one gene included in each combination is expressed among all tumor cells (340). For example, when tumor cells in which at least one gene included in the combination is expressed account for 60% or more of all of the tumor cells, the analysis apparatus may determine the corresponding combination as a target gene combination. The target gene combination includes a gene involved in the expression of a target gene recognized by CAR. At least one gene included in the gene combination is involved in the expression of one target antigen. A plurality of genes may be involved in expression of one antigen. However, here, it is assumed that each of the plurality of genes may express a different antigen.

The analysis apparatus determines the final gene combination for a target antigen among the target gene combinations (350). (i) The analysis apparatus may determine at least one of the top-rated gene combinations in terms of coverage from the target gene combinations as the final gene combination. (ii) The analysis apparatus may determine at least one of the top-rated multiple gene combinations in terms of coverage from the target gene combinations, in which coverage in normal cells is less than the reference value, as the final gene combination.

In FIG. 4, the analysis apparatus discovers a target antigen based on at least one antigen expressed in tumor cells. That is, when at least one gene in a gene combination has coverage the same as or greater than the reference value in tumor cells, the analysis apparatus may determine that the corresponding gene is involved in an effective candidate antigen.

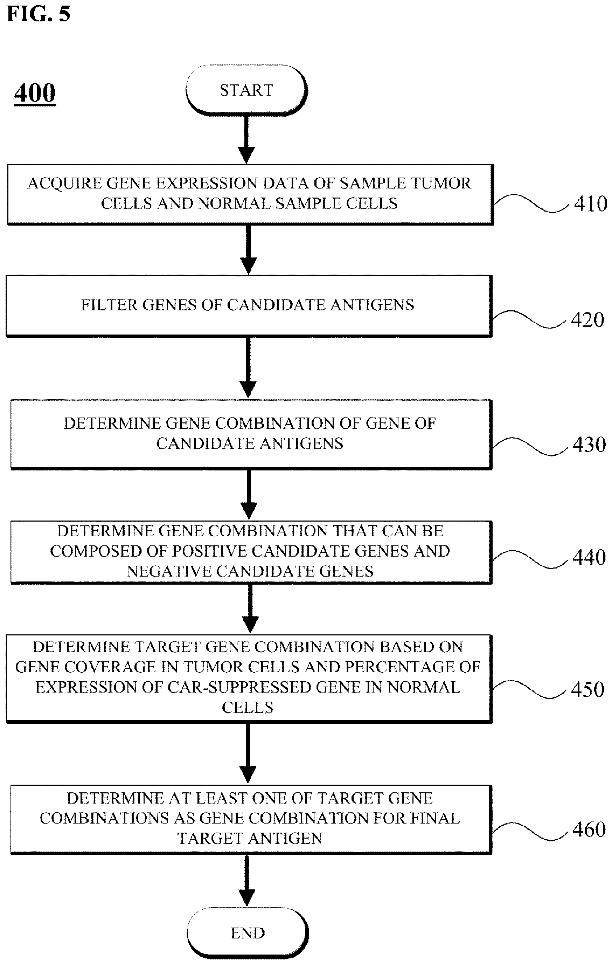

FIG. 5 is still another example of a flowchart illustrating a process (400) of discovering a target antigen for a CAR.

An analysis apparatus acquires gene expression information of sample tumor cells (410). Here, the sample may be samples of a specific type of tumor (e.g., lung cancer) collected from multiple persons. Alternatively, the sample may be cells taken from a specific patient. The analysis apparatus may also acquire gene expression information of normal sample cells (410). Information on normal cells may be used for specificity evaluation. Here, tumor cells and normal cells may be cells acquired from various cell populations, rather than a single clone.

The analysis apparatus may filter effective data from the source data (420). The analysis apparatus may select genes of candidate antigens from the source data (420).

For the candidate gene selection, a variety of criteria may be used. (i) An analysis apparatus may select a gene expressed at a certain rate or more as a candidate gene in cells included in a tumor cell group based on the tumor cell group. For example, the analysis apparatus may select a gene for an antigen expressed at 1% or more in the tumor cell group as a candidate gene. (ii) The analysis apparatus may select a gene that has a certain difference in expression pattern in tumor cells and normal cells as a candidate gene. For example, the analysis apparatus may analyze a differential expression gene (DEG) in tumor cells, compared to normal cells, as a candidate gene. The analysis apparatus may select a gene whose expression level in tumor cells is higher than the reference value compared to that of normal cells. (iii) Further, the analysis apparatus may select a candidate gene by combining the criterion (i) and the criterion (ii). For example, the analysis apparatus may select a gene differentially expressed greater than a certain standard in tumor cells among the genes for an antigen expressed 1% or greater in a tumor cell group as a candidate gene.

The analysis apparatus determines positive candidate genes and negative candidate genes in genes of the candidate antigens (430). The positive candidate gene is a gene associated with an antigen expressed tumor cell-specifically or highly in tumor cells. (i) The analysis apparatus may select a gene having coverage the same as or greater than the reference value in tumor cells among the genes of the candidate antigens as a positive candidate gene. (ii) Alternatively, the analysis apparatus may select a gene expressed in tumor cells whose number is the same as or higher than the reference value among the sample tumor cells as a positive candidate gene.

When determining the positive candidate gene, the analysis apparatus determines at least one negative candidate gene with respect to the corresponding positive candidate gene. To this end, the analysis apparatus has to possess information on the correlation in expression of genes. That is, the information on gene correlation is information on whether the expression of a specific gene promotes or inhibits the expression of at least one gene. The analysis apparatus may use transcriptome information to identify the information on the correlation between genes. The negative candidate gene is a gene whose expression pattern has a negative relationship with the positive candidate gene. That is, the negative candidate gene is a gene whose expression level is reduced when the positive candidate gene is highly expressed. The analysis apparatus may determine a negative candidate gene whose expression pattern has a negative relationship with the determined positive candidate gene. The analysis apparatus may also determine a plurality of negative candidate genes with respect to one positive candidate gene.

The analysis apparatus determines all gene combinations that can consist of positive candidate genes and negative candidate genes (440). The analysis apparatus may determine all available combinations mathematically according to the number of cases. Alternatively, the analysis apparatus may determine a possible gene combination for genes expressed substantially simultaneously considering the expression relationship of genes.

The analysis apparatus confirms whether at least one gene included in each gene combination is expressed greater than the reference rate in tumor cells. The analysis apparatus determines a target gene combination based on a rate (coverage) of cells in which at least one gene included in each combination is expressed among all tumor cells (450). Further, the analysis apparatus may determine a target gene combination based on the expression rate of a gene suppressing a CAR in normal cells (450). Several examples for the analysis apparatus determining a target gene combination will be described. (i) When tumor cells in which at least one positive candidate gene included in a combination is expressed are 60% (merely an example of the reference value) or more of all tumor cells, an analysis apparatus may determine the corresponding combination as a target gene combination. (ii) When tumor cells in which at least one negative candidate gene included in a combination is expressed are less than 10% of all tumor cells, the analysis apparatus may determine the corresponding combination as a target gene combination. (iii) When tumor cells in which at least one positive candidate gene included in a combination is expressed are 60% or more of all tumor cells, the analysis apparatus may determine the corresponding combination as a target gene combination. Here, since, during preparation, CAR-T or CAR-NK has to be designed to inhibit an immune response when a CAR binds to an antigen of a negative gene, the negative gene may be referred to as an immunosuppressive gene and serve to minimize damage to normal cells. (iv) When tumor cells in which at least one positive candidate gene included in a combination is expressed are 60% or more of all tumor cells, the analysis apparatus may determine the corresponding combination as a primary target gene combination. Afterward, the analysis apparatus may determine the primary target gene combination as a population and then as the final target gene combination. For example, among the primary target gene combinations, the analysis apparatus may determine a combination in which normal cells in which an immunosuppressive gene is expressed account for 60% or more of the normal cells in which at least one positive candidate gene included in each combination is expressed as a secondary target gene combination. The secondary target gene combination may be the final target gene combination.

The analysis apparatus finally determines a gene combination for a target antigen among the target gene combinations (460). (i) The analysis apparatus may determine at least one of the top-rated gene combinations in terms of coverage from the target gene combinations as the final gene combination. (ii) The analysis apparatus may determine at least one of the top-rated multiple gene combinations in terms of coverage from the target gene combinations, in which coverage in normal cells is less than the reference value, as the final gene combination.

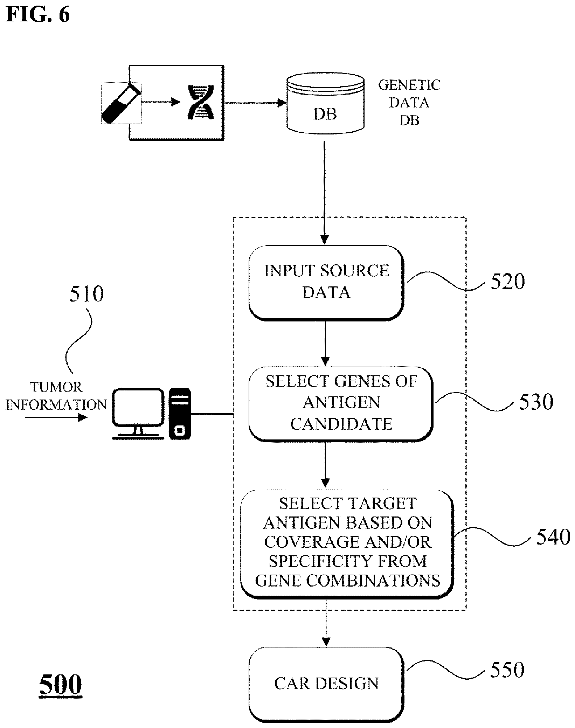

FIG. 6 is an example of a process (500) of discovering a target antigen by an analysis apparatus. The gene DB stores gene expression information of tumor cells. The gene DB may store gene expression information of normal cells. In addition, the gene DB may store information on the correlation in expression of genes.

The analysis apparatus may receive desired tumor information (510). The tumor information may include a tumor type, clinical information, and patient information. (i) The tumor information may define a specific tumor with specific clinical conditions. The clinical information may include a race, sex, and age of a sample, the stage of tumor progression (early, intermediate, late, etc.), and an anatomical area from which the sample is extracted. In this case, the tumor information will define a specific tumor with specific clinical conditions. (ii) In addition, the tumor information may define a specific tumor in a specific patient.

The analysis apparatus may input or receive source data corresponding to the tumor information received from the gene DB (520). The source data may consist of RNA expression data of tumor cells corresponding to tumor information (at least one criterion of a race, a sex, and tissue) and normal cells manipulated as above.

The analysis apparatus may select genes of antigen candidates from the source data (530). Gene filtering of an antigen candidate is as described above. Meanwhile, the process of selecting candidate genes may be omitted.

The analysis apparatus organizes a possible gene combination based on the selected genes, or genes received from the gene DB. Afterward, the analysis apparatus may select a target gene combination based on the coverage of a gene included in the corresponding combination from all possible gene combinations (540). Alternatively, the analysis apparatus may select a target gene combination based on the specificity of a gene included in the corresponding combination from all possible gene combinations (540). The processes of determining a target gene combination have been described above with reference to FIGS. 3 to 5. The analysis apparatus may select a target gene combination itself, or a more effective specific gene combination among the target gene combinations, as the final gene combination or gene for a target antigen (540).

Afterward, the analysis apparatus may deliver the final target antigen information for a CAR to another object. Alternatively, the analysis apparatus may design a recognition site for a CAR recognizing the final target antigen (550).

The *in silico* experimental processes and results for a process of discovering a target antigen based on actual genetic data will be described below.

Source data was prepared by collecting the following data. In the experimental process, the researchers used proteome analysis data for 41 cell types obtained by mass spectrometry, transcriptome data from 610 tumor cell lines, 2,769 tumor cell membrane protein genes extracted from the human surfaceome resource (Bausch-Fluck et al. PNAS, 2018) built through machine learning, proteome data and transcriptome data for 375 tumor cell lines (Nusinow et al. Cell, 2020), and 1,421 cell membrane protein genes extracted from a gene ontology database. Source data included 3,687 non-redundant genes and expression information on the corresponding genes.

As analytes, the researchers used lung cancer and colorectal cancer. Based on lung cancer LUAD and LUSC, 498 genes differentially expressed in tumor cells were selected from genes present in tumor cells and normal cells by the researchers. In addition, based on colorectal cancer COAD and READ, 315 genes differentially expressed in tumor cells were selected from genes present in tumor cells and normal cells by the researchers.

Single cell transcriptome data of lung cancer included 58 samples obtained from 44 patients. Transcriptome data of colorectal cancer included 33 samples obtained from 23 patients. Tumor epithelial cells were isolated through single cell transcriptome analysis technology, and other immune cells were considered normal cells. Lung cancer cells consisted of 165,511 tumor cells and 42,995 normal cells, and colorectal cancer cells consisted of 47,285 tumor cells and 16,404 normal cells.

Hereinafter, the process expressed as being conducted by the "researchers" is a process performed by all researchers using a computer device, such as an "analysis apparatus."

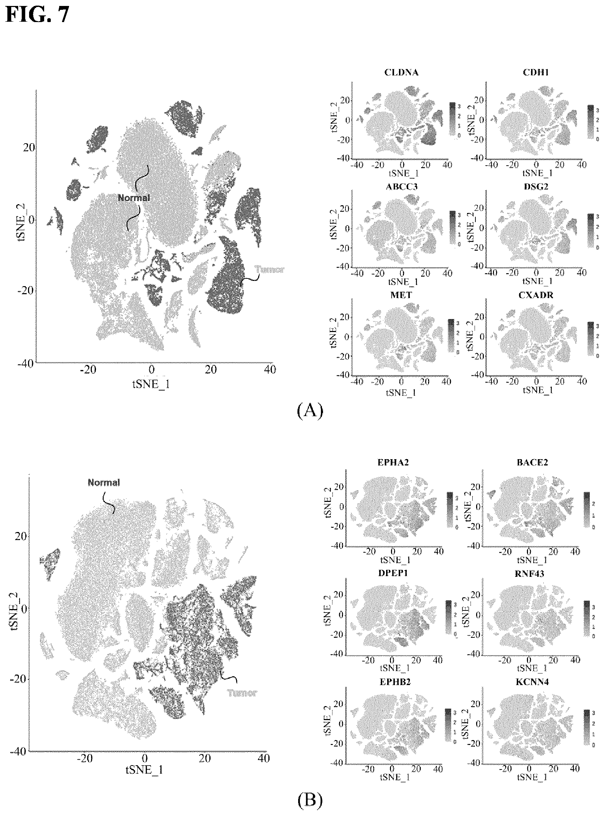

Genes of candidate antigens were first filtered from the source data by the researchers. Genes exhibiting at least 1% or more gene expression (cell coverage) in tumor cells were selected through single cell analysis by the researchers. In addition, genes whose expression level itself showed a difference of the reference value or greater were selected through DEG analysis between normal cells and tumor cells by the researchers. Finally, 163 genes of lung cancer and 110 genes of colorectal cancer were secured by the researchers. FIG. 7 is an experimental result showing the gene expression patterns of tumor cells and normal cells in a colorectal cancer sample and a lung cancer sample. FIG. 7 is the t-stochastic neighbor embedding (SNE) results, expressing the gene expression patterns through dimensionality reduction. FIG. 7A is the expression pattern of 163 lung cancer genes. The left panel of FIG. 7A shows a difference in expression between tumor cells and normal cells based on entire genes, and the right panel of FIG. 7A shows an expression difference based on a specific gene. FIG. 7B is the expression patterns of 110 genes. The left panel of FIG. 7B shows a difference in expression between tumor cells and normal cells based on entire genes, and the right panel of FIG. 7B shows an expression difference based on a specific gene.

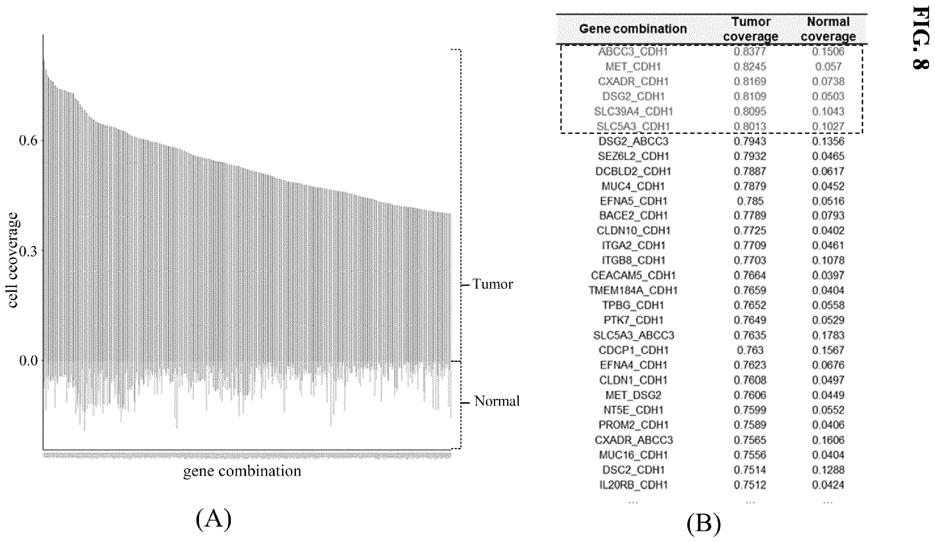

From 163 lung cancer genes, 70 genes having coverage in tumor cells of 10% or more, except `CLDN4 (93.5%)' genes having coverage in tumor cells of 90% or more, were selected by the researchers. 2,415 gene combinations were determined using 70 genes by the researchers. Whether the gene expression was in a single cell unit, in which at least one of the genes included in each gene combination was expressed, was converted into binary information by the researchers. 253 combinations having coverage in tumor cells of 60% or more were selected by the researchers. FIG. 8 is an experimental result for a gene combination in which one or more genes are expressed in a lung cancer sample. FIG. 8A shows the expression rates of 253 gene combinations in tumor cells and normal cells. FIG. 8B shows tumor combinations having relatively high coverage in tumor cells, and the top-rated combinations having the coverage of 80% or more are indicated by dotted boxes. In this case, the analysis apparatus may select the gene combination having the highest coverage in tumor cells among the top-rated combinations as a final antigen candidate. In addition, the analysis apparatus may select the gene combination having low coverage in normal cells among the gene combinations having high coverage in tumor cells as a final antigen candidate. In summary, the analysis apparatus may select the final antigen candidate based on at least one of the coverage in tumor cells and the coverage in normal cells.

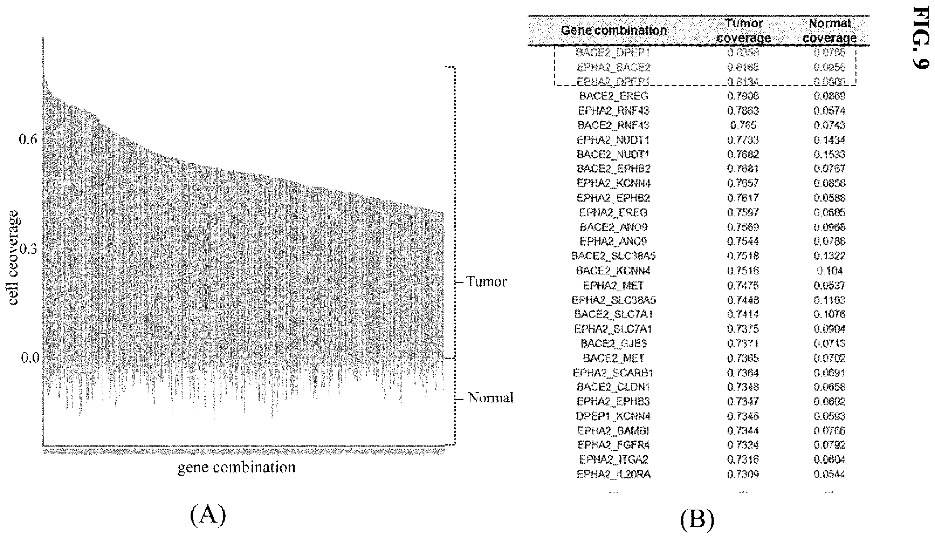

Genes having coverage in tumor cells of 10% or more were selected from 110 colorectal cancer genes by the researchers. 2,278 gene combinations were determined using the selected genes by the researchers. Whether the gene expression was in a single cell unit, in which at least one of the genes included in each gene combination was expressed, was converted into binary information by the researchers. 248 combinations having coverage in tumor cells of 60% or more were selected by the researchers. FIG. 9 is an experimental result for a gene combination in which one or more genes are expressed in a colorectal cancer sample. FIG. 9A shows the expression rates of 248 gene combinations in tumor cells and normal cells. FIG. 9B shows the gene combinations having relatively high coverage in tumor cells, and the top-rated combinations having the coverage of 80% or more are indicated by dotted boxes. In this case, the analysis apparatus may select the gene combination having the highest coverage in tumor cells among the top-rated combinations as a final antigen candidate. In addition, the analysis apparatus may select the gene combination having low coverage in normal cells among the gene combinations having high coverage in tumor cells as a final antigen candidate. In summary, the analysis apparatus may select the final antigen candidate based on at least one of the coverage in tumor cells and the coverage in normal cells.

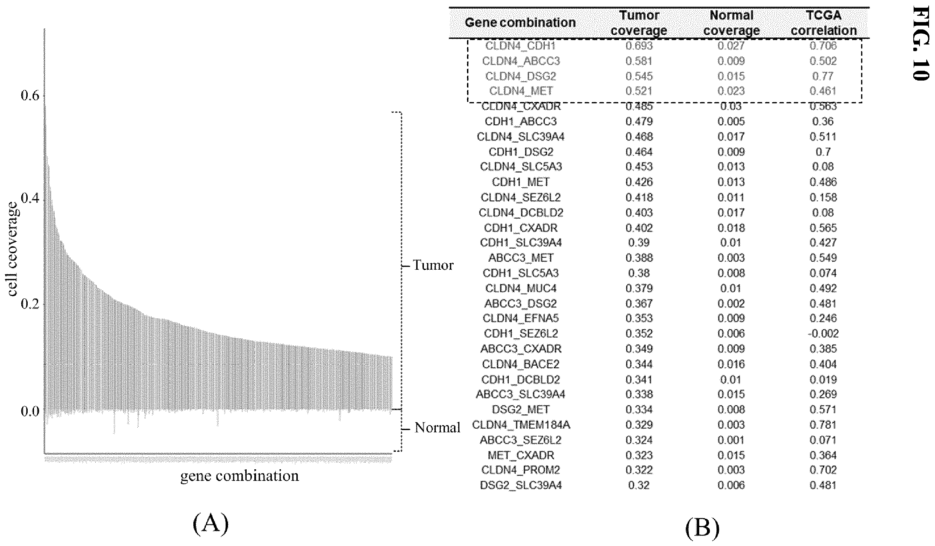

Genes having coverage in tumor cells of 10% or more were selected from 163 lung cancer genes by the researchers. 2,415 gene combinations were determined using the selected genes by the researchers. Whether the gene expression was in a single cell unit, in which two or more genes included in each gene combination were simultaneously expressed, was converted into binary information by the researchers. 589 combinations having coverage in tumor cells of 10% or more were selected by the researchers. FIG. 10 is an experimental result for a gene combination in which two or more genes are expressed in a lung cancer sample. FIG. 10A shows the expression rates of 589 gene combinations in tumor cells and normal cells. FIG. 10B shows tumor combinations having relatively high coverage in tumor cells, and the top-rated combinations having the coverage of 50% or more are indicated by dotted boxes. In this case, the analysis apparatus may select the gene combination having the highest coverage in tumor cells among the top-rated combinations as a final antigen candidate. In addition, the analysis apparatus may select the gene combination having low coverage in normal cells among the gene combinations having high coverage in tumor cells as a final antigen candidate. In addition, the analysis apparatus may select the gene combination having a high correlation in expression between two or more co-expressed genes. In summary, the analysis apparatus may select the final antigen candidate based on at least one of coverage in tumor cells, coverage in normal cells, and the correlation in expression of genes.

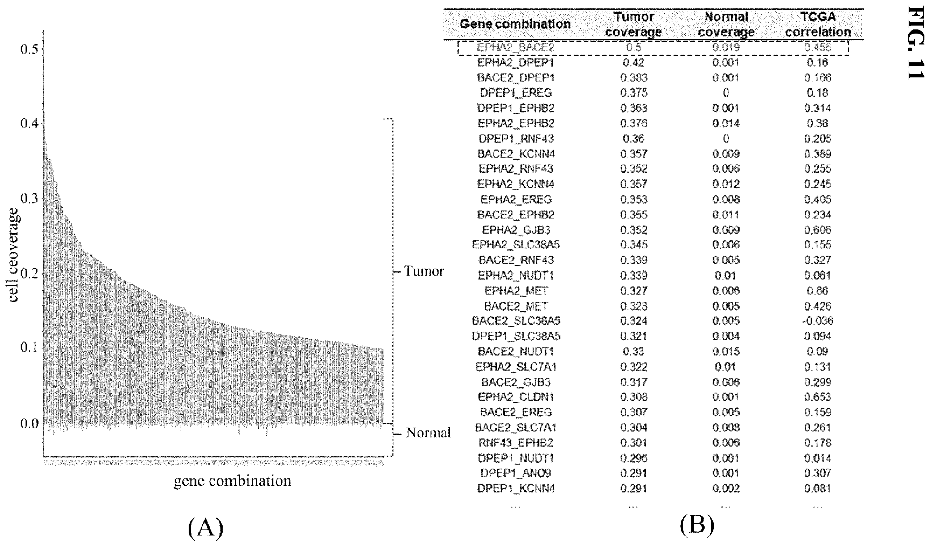

Genes having coverage in tumor cells of 10% or more were selected from 110 colorectal cancer genes by the researchers. 2,278 gene combinations were determined using the selected genes by the researchers. Whether the gene expression was in a single cell unit, in which at least one of the genes included in each gene combination was expressed, was converted into binary information by the researchers. 537 combinations having coverage in tumor cells of 60% or more were selected by the researchers. FIG. 11 is an experimental result for a gene combination in which two or more genes are expressed in a colorectal cancer sample. FIG. 11A shows the expression rates of 537 gene combinations in tumor cells and normal cells. FIG. 11B shows the gene combinations having a relatively high coverage in tumor cells, and the top-rated combinations having the coverage of 50% or more are indicated by dotted boxes. In this case, the analysis apparatus may select the gene combination having the highest coverage in tumor cells among the top-rated combinations as a final antigen candidate. In addition, the analysis apparatus may select the gene combination having low coverage in normal cells among the gene combinations having high coverage in tumor cells as a final antigen candidate. In addition, the analysis apparatus may select the gene combination having a high correlation in expression between two or more co-expressed genes. In summary, the analysis apparatus may select the final antigen candidate based on at least one of coverage in tumor cells, coverage in normal cells, and the correlation in expression of genes.

All co-expression indexes between gene combinations in tumors were calculated using 10,459 transcriptome sequence data obtained from a TCGA DB by the researchers. Possible combinations were drawn from genes (negative candidate genes) having a negative correlation with six main candidate genes (positive candidate genes), selected from lung cancer and colorectal cancer cells by the researchers. A protection rate was used to evaluate the specificity of a CAR by the researchers. The protection rate is a rate of expressing a tumor cell-specific gene (positive gene), together with an immunosuppressive gene (negative gene) having a suppressive action on a CAR, in normal cells. Here, during preparation, CAR-T or CAR-NK has to be designed to inhibit an immune response when a CAR binds to an antigen and may serve to minimize damage to normal cells. For the suppressive gene, the researchers' own experimental results or results from a previous study may be used according to the characteristics of a CAR.

Main positive candidate genes were selected from lung cancer genes by the researchers. As the main positive candidate genes, six genes having high coverage were selected based on coverage in tumor cells. In addition, the researchers selected negative candidate genes whose expression pattern had a negative relationship with the positive candidate genes. One or more negative candidate genes may be selected for one positive candidate gene. From all gene combinations that can consist of the selected positive candidate genes and negative candidate genes, 462 combinations having coverage in tumor cells of 60% or more were selected by the researchers.

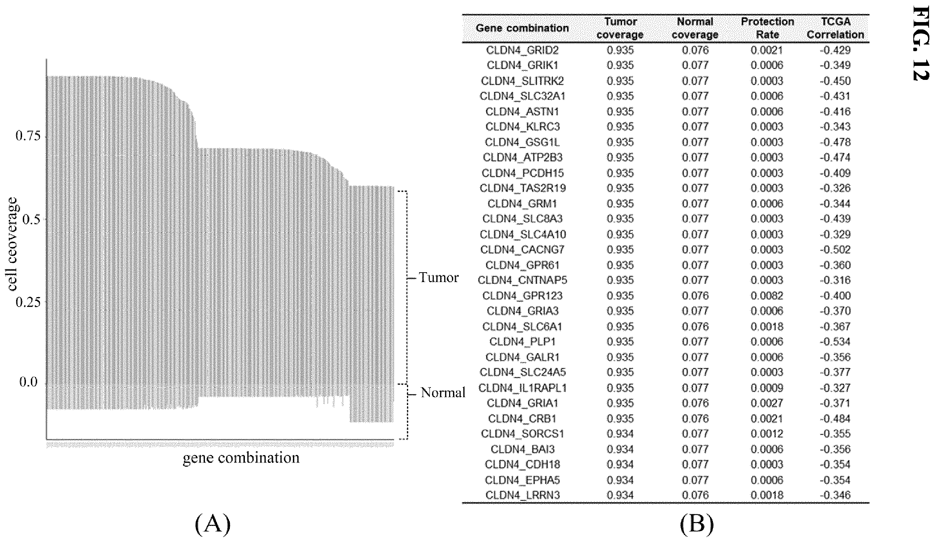

FIG. 12 is an experimental result for a tumor cell-specific gene combination in a lung cancer sample. FIG. 12A shows the expression rates of 462 gene combinations in tumor cells and normal cells. In FIG. 12B, gene combinations having relatively high coverage in tumor cells are indicated. FIG. 12B shows values of items, such as coverage in normal cells (tumor coverage), coverage in normal cells (normal coverage), a protection rate, and the correlation in expression of genes included in a gene combination (TCGA correlation). In this case, the analysis apparatus may select a gene combination having tumor coverage higher than a certain value as a final antigen candidate. In addition, the analysis apparatus may select a gene combination having normal coverage lower than a certain level among gene combinations having high tumor coverage as a final antigen candidate. In addition, the analysis apparatus may select a gene combination having a protection rate higher than a certain level among gene combinations having high tumor coverage as a final antigen candidate. In addition, the analysis apparatus may select a gene combination having a high correlation in expression between a positive candidate gene and a negative candidate gene as a final antigen candidate. In summary, the analysis apparatus may select a final antigen candidate based on a combination of tumor coverage, normal coverage, a protection rate, and the correlation in expression of genes.

Main positive candidate genes were selected from colorectal cancer genes by the researchers. As the main positive candidate genes, six genes having high coverage were selected based on tumor coverage. In addition, negative candidate genes whose expression pattern had a negative relationship with each positive candidate gene were selected by the researchers. One or more negative candidate genes may be selected for one positive candidate gene. From all gene combinations which can consist of the selected positive candidate genes and negative candidate genes, 449 combinations having tumor coverage of 40% or more were selected by the researchers.

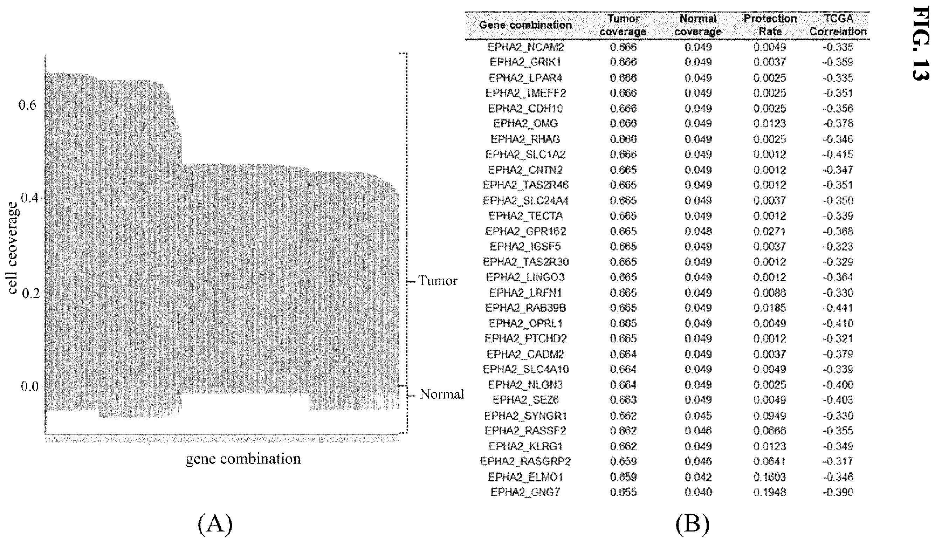

FIG. 13 is an experimental result for a tumor cell-specific gene combination in a colorectal cancer sample. FIG. 13A shows the expression rates of 462 gene combinations in tumor cells and normal cells. In FIG. 13B, gene combinations having relatively high coverage in tumor cells are indicated. FIG. 13B shows values of items, such as coverage in tumor cells (tumor coverage), coverage in normal cells (normal coverage), a protection rate, and the correlation in expression of genes included in a gene combination (TCGA correlation). In this case, the analysis apparatus may select a gene combination having tumor coverage higher than a certain value as a final antigen candidate. In addition, the analysis apparatus may select a gene combination having normal coverage lower than a certain value among the gene combinations having high tumor coverage as a final antigen candidate. In addition, the analysis apparatus may select a gene combination having a protection rate higher than a certain value among the gene combinations having high tumor coverage as a final antigen candidate. In addition, the analysis apparatus may select a gene combination having a high correlation in expression between a positive candidate gene and a negative candidate gene as a final antigen candidate. In summary, the analysis apparatus may select a final antigen candidate based on a combination of tumor coverage, normal coverage, a protection rate and the correlation in expression of genes.

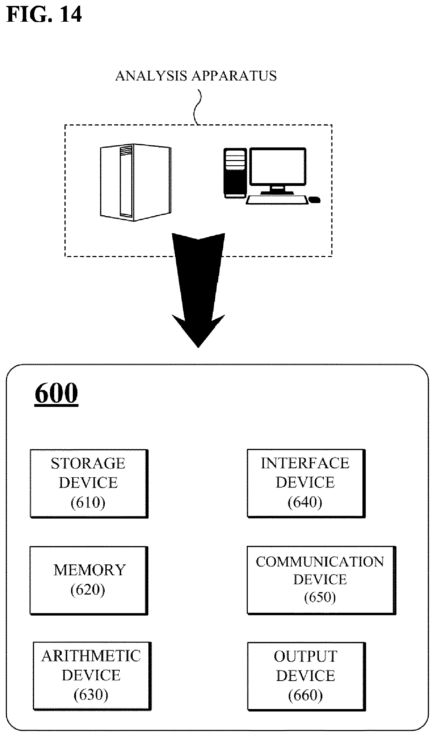

FIG. 14 is an example of an analysis apparatus for discovering an antigen for a CAR.

An analysis apparatus 600 is an apparatus that corresponds to the analysis apparatus 130 or 140 in FIG. 1. The analysis apparatus 600 discovers a target antigen recognized by a CAR.

The analysis apparatus 600 may be realized in various physical forms. For example, the analysis apparatus 600 may have a form such as a computer device like PC, a network server, or a data processing-exclusive chipset. Computer devices may include a mobile device such as a smart device.

The analysis apparatus 600 may include a storage device 610, a memory 620, an arithmetic device 630, an interface device 640, a communication device 650, and an output device 660.

The storage device 610 stores a program for determining an antigen recognized by a CAR using expression information of genes in tumor cells. The corresponding program may determine an antigen recognized by a CAR using gene expression information in not only tumor cells but also normal cells. Further, the storage device 610 may store programs or source codes necessary for data processing. The storage device 610 may store input genetic data and/or tumor information necessary for analysis.

The memory 620 may store data and information, which are produced in the process of analyzing the genetic data by the analysis apparatus 600.

The interface device 640 is an apparatus that receives certain commands and data from the outside. The interface device 640 may receive genetic data from a physically connected input device or external storage device. The interface device 640 may receive expression data of genes in tumor cells and normal cells in a single cell device. The interface device 640 may also receive analysis criteria, tumor information, etc.

The communication device 650 is a component that receives and transmits certain information via a wired or wireless network. The communication device 650 may receive genetic data from an external object. The communication device 650 may receive expression data of genes in tumor cells and normal cells in a single cell device. The communication device 650 may transmit target antigen-related information, which is an analysis result, to an external object.

The communication device 650 or the interface device 640 is an apparatus that receives certain data or commands from the outside. The communication device 650 or the interface device 640 may be referred to as an input device.

The output device 660 is an apparatus that outputs certain data. The output device 660 may output an interface, an analysis result, etc., necessary for data processing.

The arithmetic device 630 may discover a target antigen by analyzing genetic data using the program stored in the storage device 610.

The arithmetic device 630 may select genes of candidate antigens (candidate genes) from source data. The criteria and procedure for candidate gene selection are as follows.

The arithmetic device 630 may determine all initial gene combinations which can consist of a plurality of genes among the genes of the candidate antigens. The arithmetic device 630 may determine a target gene combination associated with a target antigen from the initial gene combinations. In addition, the arithmetic device 630 may select an optimal final gene combination or gene when there are a plurality of gene combinations in the target gene combinations.

The arithmetic device 630 may select a combination in which a rate (coverage) of cells in which at least one gene included in an initial gene combination is expressed among tumor cells is the same as or greater than the reference value as a target gene combination.

The arithmetic device 630 may select a combination in which a rate (coverage) of cells in which a plurality of genes included in an initial gene combination is expressed among tumor cells is the same as or greater than the reference value as a target gene combination.

The arithmetic device 630 may determine a positive candidate gene from genes of candidate antigens based on the percentage of cells in which a gene is expressed in tumor cells or the number of cells in which a gene is expressed in tumor cells. The arithmetic device 630 may determine negative candidate genes whose expression pattern has a reverse relationship with the positive candidate genes.

The arithmetic device 630 may determine all gene combinations that can consist of positive candidate genes and negative candidate genes as an initial gene combination.

The arithmetic device 630 may determine a combination in which the percentage of cells in which at least one positive candidate gene of the positive candidate genes included in a specific combination is expressed among tumor cells is the same as or greater than the reference value, and
the percentage of normal cells in which a suppressive gene suppressing CAR recognition or linkage with a positive candidate gene-involved antigen among normal cells in which at least one positive candidate gene is expressed is the same as or greater than the reference value as a target gene combination.

The arithmetic device 630 may be an apparatus for processing data and certain calculations, such as a processor, AP, or a program-embedded chip.

In addition, the target antigen discovery method for a CAR as described above may be implemented by a program (or application) including an executed algorithm that can be run by a computer. The program may be provided by being stored in a transitory or non-transitory computer readable medium.

The non-transitory computer readable medium is a medium that stores data semi-permanently and is able to be read by an apparatus, rather than a medium that stores data temporarily, such as a register, a cache, or a memory. Specifically, the above-described various applications or programs may be provided by being stored in a non-transitory computer readable medium such as a CD, DVD, hard disk, Blu-ray disc, USB, memory card, read-only memory (ROM), programmable read-only memory (PROM), erasable PROM (EPROM), electrically erasable PROM (EEPROM), or flash memory.

The transitory computer readable medium includes various RAMs, such as a static RAM (RAM), dynamic RAM (DRAM), synchronous DRAM (SDRAM), double data Rate SDRAM (DDR SDRAM), enhanced SDRAM (ESDRAM), Synclink DRAM (SLDRAM), and direct Rambus RAM (DRRAM).

The present embodiments and the accompanying drawings in the specification merely clearly show parts of the technical ideas included in the above-described technology, and it should be apparent that all modifications and specific examples that can be easily inferred by those of ordinary skill in the art included in the specification and drawings of the above-described technology are included in the scope of rights of the above-described technology.

## Claims

1. A target antigen discovery method for a chimeric antigen receptor (CAR), comprising:
acquiring expression information of genes in tumor cells in a single cell unit by an analysis apparatus;
extracting genes of candidate antigens having an expression level of a reference value or higher from the genes by the analysis apparatus;
determining gene combinations which can consist of the genes of the candidate antigens by the analysis apparatus;
determining a target gene combination in which at least one gene included in the combination is expressed among the gene combinations by the analysis apparatus; and
determining at least one of the target gene compositions as a gene combination for a target antigen by the analysis apparatus,
wherein the at least one gene is a gene for an antigen in the tumor cells, recognized by the CAR, and the target gene combination is a gene combination in which the percentage of cells in which the at least one gene is expressed in tumor cells is the reference value or more.

2. The method of claim 1, wherein the analysis apparatus extracts genes in which an expressing percentage in the tumor cells is the reference value or more among genes of the tumor cells as genes of the candidate antigens.

3. The method of claim 1, wherein the analysis apparatus extracts genes in which a difference in expression level in normal cells and the tumor cells is the reference value or more among genes of the tumor cells as genes of the candidate antigens.

4. The method of claim 1, wherein the analysis apparatus determines the target gene combination in which a plurality of genes comprised therein are expressed from the gene combinations in which the target gene combination is a gene combination in which the percentage of cells in which the plurality of genes are simultaneously expressed among the tumor cells is the reference value or more.

5. The method of claim 4, wherein the plurality of genes are involved in expression of different antigens.

6. A target antigen discovery method for a chimeric antigen receptor (CAR), comprising:
acquiring expression information of genes of tumor cells in a single cell unit by an analysis apparatus;
extracting genes of candidate antigens having an expression level of a reference value or higher from the genes by the analysis apparatus;
determining positive candidate genes from genes of the candidate antigens by the analysis apparatus, based on the percentage of expressing cells in the tumor cells or the number of expressing cells in the tumor cells;
determining negative candidate genes whose expression pattern has a reverse relationship with the positive candidate genes among genes of the candidate antigens by the analysis apparatus;
determining gene combinations that can be composed of the positive candidate genes and the negative candidate genes by the analysis apparatus;
determining a target gene combination for an antigen recognized by the CAR among the gene combinations by the analysis apparatus, based on the percentage of cells in which at least one positive candidate gene included in a combination of the gene combinations is expressed among the tumor cells and the percentage of cells in which at least one negative candidate gene of the negative candidate genes inhibiting an immune response of CAR cells for an antigen produced by expressing the at least one positive candidate gene is expressed among normal cells; and
determining at least one combination of the target gene combinations as a gene combination for a target antigen by the analysis apparatus.

7. The method of claim 6, wherein the analysis apparatus extracts genes in which the percentage of expressing cells in the tumor cells is the reference value or more among genes of the tumor cells as genes of the candidate antigens.

8. The method of claim 6, wherein the analysis apparatus extracts genes in which a difference in expression level in normal cells and the tumor cells is the reference value or more among genes of the tumor cells as genes of the candidate antigens.

9. The method of claim 6, wherein the analysis apparatus determines the specific combination in which the percentage of tumor cells in which at least one positive candidate gene of the positive candidate genes included in a specific combination is expressed among the tumor cells is the reference value or more, and
the percentage of normal cells in which a suppressive gene suppressing immune responses of an antigen and the CAR by the positive candidate gene is expressed among normal cells in which at least one positive candidate gene is expressed is the reference value or more as the target gene combination.

10. The method of claim 6, wherein the positive candidate genes are involved in expression of different antigens.

11. An analysis apparatus for designing a chimeric antigen receptor (CAR), comprising:
an input device for receiving expression data of genes in tumor cells of a sample in a single cell unit;
a storage device for storing a program that determines an antigen recognized by the CAR using the expression information of genes in tumor cells; and
an arithmetic device for determining a target gene combination or target gene for a target antigen of tumor cells in the sample recognized by the CAR from gene combinations that can consist of a plurality of genes among the genes of the candidate antigens determined from the expression data of the genes,
wherein the target gene combination has the percentage of cells in which at least one gene included therein is expressed among the tumor cells of the reference value or more.

12. The analysis apparatus of claim 11, wherein genes of the candidate antigens are genes in which the percentage of expressing cells in the tumor cells is a first reference value or more among genes of the tumor cells.

13. The analysis apparatus of claim 11, wherein genes of the candidate antigens are genes in which a difference in expression level in normal cells and the tumor cells is the reference value or more among genes of the tumor cells.

14. The analysis apparatus of claim 12, wherein the plurality of genes have the percentage of expressing cells in the tumor cells of a second reference value or more among genes of the candidate antigens, and the second reference value is a value higher than the first reference value.

15. The analysis apparatus of claim 11, wherein the target gene combination has the percentage of cells in which at least a plurality of genes are expressed included in the target gene combination among the tumor cells of the reference value or more.

16. The analysis apparatus of claim 11, wherein the input device further receives expression data of genes of normal cells of the sample,
the arithmetic device determines positive candidate genes and negative candidate genes whose expression pattern has a reverse relationship with the positive candidate genes from genes of the candidate antigens, based on the percentage of expressing cells in tumor cells or the number of expressing cells in the tumor cells, and
all gene combinations that are able to be composed of the positive candidate genes and the negative candidate genes are determined as the gene combinations.

17. The analysis apparatus of claim 16, wherein the arithmetic device determines a specific combination in which the percentage of cells in which at least one positive candidate gene among the positive candidate genes included in the specific combination is expressed among the tumor cells is the reference value or more, and
the percentage of normal cells in which a suppressive gene suppressing recognition or binding of an antigen or the CAR, involving the positive candidate gene, among normal cells in which the at least one positive candidate gene is expressed is the reference value or more as the target gene combination.
